# EUROPEAN PATENT APPLICATION

(11) **EP 4 036 925 A1**
(43) Date of publication of application: **03.08.2022**
(21) Application number: 19947308.3
(22) Date of filing: 24.09.2019
(51) Int. Cl.: G16H 20/30

(54) **EXPIRATORY PRESSURE LOAD TRAINING MANAGEMENT DEVICE AND SYSTEM**

(71) Applicant: Respiratory Science Co., Ltd., Toyonaka-shi, Osaka, 560-0045 (JP)
(72) Inventor: MIKI Keisuke, Toyonaka-shi, Osaka 560-0045 (JP)
(74) Representative: Casalonga
(86) International application number: PCT/JP2019/037405
(87) International publication number: WO 2021/059354

(57) **Abstract**

Provided is an expiratory pressure load training management device for reducing exertional dyspnoea of a patient with chronic obstructive pulmonary disease (COPD) and improving exercise tolerance and physical activity. The device comprises: a means for inputting exhalation information relating to an inhalation or exhalation time ratio T_{EX} for one breathing period during maximum exertion by a user in cardiopulmonary exercise testing (CPET), and an inhalation or exhalation time ratio T_{RE} for one breathing period at rest time according to an examination; a means for calculating the difference between T_{EX} and T_{RE} and determining whether there was prolonged exhalation by the user during exercise; a means for presenting, to a user who was determined to have prolonged exhalation during exercise, a menu of expiratory pressure load training based on a maximum expiratory muscle strength value; and a means for inputting exhalation information after a prescribed number of cycles of the expiratory pressure load training, and information relating to exercise tolerance, judging whether there was an improvement in exercise tolerance of the target user, and presenting training results.

## Description

### [Technical Field]

The present disclosure relates to a technique for expiratory pressure load training management which contributes to improving dyspnoea in chronic obstructive pulmonary disease (COPD).

### [Background Art]

COPD is a lifestyle-related disease in which the lungs are persistently inflamed by long-term exposure to toxic substances contained in cigarette smoke and the like, resulting in reduced pulmonary function. World Health Organization estimates in 2018 indicated that COPD was the third leading cause of death worldwide, with 65 million people worldwide suffering from moderate or severe COPD. According to NICE Study (published in 2001), which is a report on epidemiological studies by Mr. Fukuchi and others at Juntendo University of Medicine, about 5.3 million people in Japan suffer from COPD. Recently, medical, economic and social measures against COPD have become an urgent necessity due to soaring social security benefit costs and national health care costs. Improving dyspnoea, which is the most common complaint in patients with COPD, is an urgent task. No improvement in exercise tolerance or physical activity is possible without relief of dyspnoea.

β² stimulants and anticholinergics are the mainstream of known long-acting inhaled drugs prescribed to relieve symptoms of COPD. Though non-pharmacotherapy by the pulmonary rehabilitation is now available in addition to many other drugs, it is actual that the treatment aggression of COPD has not been reached. In order to improve dyspnoea, it is necessary to grasp the pathophysiology of the diverse COPD, and then to take measures suitable for it in a tailored manner.

In view of the aging society, the present inventor believes that it is more necessary to provide exercise opportunities such as pulmonary rehabilitation, nutritional therapy, and walking, which are not satisfactory in daily practice. The present inventor is a respiratory specialist and has many opportunities to contact patients with COPD. The meaning of "it is easier and more comfortable to take a quick and forceful breath, rather than pursed-lip breathing during exercise, and with such a comfortable timing of breathing," which was described as the actual sensation of COPD, was difficult for the inventor to understand by the conventional medical common sense that recommends pursed-lip breathing, and motivated the inventor to explore ways to improve exertional dyspnoea.

The present inventor has intensively studied the relationship among three variables related to exertional dyspnoea: the exercise tolerance of COPD, the timings of inhalation and exhalation, and the balance of respiratory muscle strength. As a result, it was found that the expiratory muscle strength was insufficient in the breathing pattern in which the exhalation was prolonged and the respiratory frequency was not increased with reduce tidal volume (hereinafter also referred to as "slow-shallow pattern"), and the exercise tolerance was reduced. Conventionally, there has been no report mentioned about the usefulness of expiratory muscles training (EMT) for actual COPD subjects, but the present inventor has hypothesized that if EMT increases sufficient breath with the improved muscles strength among laryngeal muscles, adequate inhalation is stimulated, leading to improved exercise tolerance with reduced dyspnoea (Non-Patent Document 1).

On the other hand, breathing patterns with no prolonged exhalation and high respiratory frequency with reduce tidal volume (hereinafter also referred to as "rapid-shallow") showed dyspnoea when tachypnea occurred, although exercise tolerance was relatively maintained. Although the effectiveness of inspiratory muscle training (IMT), which has recently attracted attention, is not necessarily constant, the present inventor hypothesized that rapid-shallow respiratory patterns would lead to improved dyspnoea if adequate inhalation was obtained by IMT (Non-Patent Document 1).

### [Prior Art]

### [Non-patent Document]

[Non-patent Document 1] Keisuke MIKI, "Exercise tolerance and balance of inspiratory-to-expiratory muscle strength in relation to breathing timing in patients with chronic obstructive pulmonary disease", Journal of Breath Research, 2018 Mar 28;12 (3) .

### [Outline of the Invention]

### [Problems to be Solved by the Invention]

As mentioned above, there is a problem of increasing the number of people suffering from COPD, and in order to cope with this problem, there is a need to widely disseminate training useful for improving dyspnoea, exercise tolerance, and physical activity, which is the most frequent complaint of COPD patients.

However, the effectiveness of inspiratory muscle training (IMT), the efficacy of which has been noticed, is not necessarily constant, and there are no reports mentioning the usefulness of expiratory muscle training (EMT) for actual COPD patients.

In view of the above circumstances, it is an object of the present invention to provide an expiratory pressure load training management device, management system, and management method useful for an actual COPD patient in order to reduce dyspnoea of a COPD patient and improve exercise tolerance and physical activity of the patient.

### [Means to Solve the Objects]

The present inventor conducted a clinical study on the effectiveness of expiratory muscle training (EMT) in COPD patients, conducted the treatment strategy that EMT could be more effective in COPD patients when expiratory prolongation patterns are confirmed by a decreased ratio of inspiratory time to total respiratory cycle time during exercise (slow-shallow breathing pattern), whereas inspiratory muscle training (IMT) could be more effective when non-expiratory prolongation patterns are confirmed by increased ratio of inspiratory time to total respiratory cycle time during exercise (rapid-shallow breathing pattern). Thereafter, the present inventor evaluated and confirmed the usefulness of each, and completed the present invention.

In order to achieve the above object, the expiratory pressure load training management device is a training management device for a user having chronic obstructive pulmonary disorder (COPD), and comprises the following measures 1) to 4).
1) An exhalation information input means for inputting exhalation information on the time ratio T_{EX} of inhalation or the time ratio T_{EX} of exhalation to total respiratory cycle time at peak exercise during cardiopulmonary exercise testing (CPET) for a user, and the time ratio T_{RE} of inhalation or of exhalation to total respiratory cycle time at rest during CPET.
2) A user determination means for calculating a difference between T_{EX} and T_{RE}, and determining whether or not the user has exhalation prolongation during exercise.
3) A menu presenting means for presenting a menu of expiratory pressure load training based on a maximum expiratory muscle strength value to a user determined to have exhalation prolongation during exercise.
4) A training result presenting means for inputting the exhalation information and information related to exercise tolerance in 1) above after a predetermined number of training sessions, and presenting a training result to determine whether or not there is an improvement effect of exercise tolerance of the user.

According to the device having the above configuration, it is possible to reduce dyspnoea and improve exercise tolerance and physical activity for actual COPD patients.

Here, a cardiopulmonary exercise testing (CPET) is also called CPX, and is a test in which exercise load test is performed using exercise load devices such as a treadmill and a cycle ergometer (bicycle), and at the same time, O₂ concentration, CO₂ concentration, and ventilatory flow in exhaled breath are measured in real time by an expiratory gas analyzer, and an index such as peak oxygen uptake which is an index of exercise tolerance is measured. CPET is a valuable test for assessing the motor pathophysiology of individual patients and can measure inspiratory tidal volume and expiratory tidal volume in a single breath.

Also, the lower the ratio of inspiratory time to single total breath time, the longer the exhalation. The exhalation information inputted to the exhalation information input means is the inhalation time ratio for one breath time at rest during CPET for T_{RE} when T_{EX} is the inhalation time ratio for one breath time at peak exercise, and the exhalation time ratio for one breath time at rest during CPET for T_{RE} when T_{EX} is the exhalation time ratio for one breath time at peak exercise. In other words, if the inhalation time ratio is used, the same breathing phase evaluation, that is, the inhalation time ratio should be used for both T_{EX} and T_{RE}, and if the exhalation time ratio is used, the same exhalation time ratio should be used for both T_{EX} and T_{RE}. Thus, for example, if T_{EX} is the ratio of inspiratory time to single breath time during maximal exercise, T_{RE} will not be the ratio of expiratory time to single breath time at rest by examination.

The user determination means calculates a difference between T_{EX} and T_{RE}, and, for example, when T_{EX} and T_{RE} are used as a ratio of the inhalation time, calculates a value of T_{EX}-T_{RE}, determines whether or not the difference value is a negative value, and determines that a user who is a negative value is a user who causes an exhalation prolongation during exercise. Then, the menu presenting means provides a menu of expiratory pressure load training for a user who is determined to have an expiratory prolongation during exercise, and prompts the user to perform the expiratory pressure load training.

The user determination means calculates a difference between T_{EX} and T_{RE}, and, for example, calculates a value of T_{EX} - T_{RE} when T_{EX} and T_{RE} are used as a ratio of the inhalation time, determines whether or not the difference value is a positive value, and determines that a user who is a positive value is a user who does not cause expiratory prolongation during exercise. The menu presenting means provides a menu of inspiratory pressure load training for a user who is determined not to have an expiratory prolongation during exercise, and prompts the user to perform the inspiratory pressure load training.

In the expiratory pressure load training management device of the present invention, the expiratory pressure load training preferably uses an expiratory pressure load device with a one-way valve, and the expiratory pressure load device functions to increase the expiratory muscle strength among laryngeal muscles by opening the valve when the set pressure is reached and exhaling air flows in.

For example, manufactured by EMST150 (Aspire Products LLC Co., Ltd.) can be used as the expiratory pressure loading device. Currently, EMST150 is not sold in Japan, but only overseas.

The set pressure in the expiratory pressure load device described above can be set at a set range of low pressure below 30cmH₂O, and can be trained and increased in a stepwise manner from a lower expiratory pressure load of 20% to 30% of based on the lower maximum expiratory muscle strength value of the users. To the best of the inventors' knowledge, conventional expiratory pressure loading devices do not provide optimal pressure settings for at least Japanese COPD patients. For EMST150, there is no low-pressure setting function below 30cmH₂O. In order to realize respiratory muscle training in which expiratory pressure load training is tailored for each user by the pathophysiology, it is preferable to enable setting below 30cmH₂O.

It is also possible to minimize muscle pain and exacerbation of dyspnoea from respiratory muscle fatigue due to training by starting training from a relatively low expiratory pressure load of 20% to 30% of the user's maximum expiratory muscle strength.

Since the respiratory pressure load training can be easily carried out even in the elderly at home, the training is carried out at the load pressure suitable for each user, and the muscle pain and the aggravation of the dyspnoea from the respiratory muscle fatigue due to the training are minimized, and the user is continued so as not to cause an excessive load.

The expiratory pressure load training management device of the present invention presents a menu of the inspiratory pressure load training in the menu presenting means when there is no exhalation prolongation during exercise as a result of calculating the difference in the user determination means.

As a result, it is possible to realize tailored respiratory pressure load training for selecting EMT and IMT by pathophysiology.

The training menu in the menu presenting means of the expiratory pressure load training management device of the present invention is preferably confirmed and approved by the respiratory specialist. The involvement of the respiratory specialist in expiratory pressure load training allows the user to provide appropriate training menus from medical knowledge. In some cases, during expiratory pressure load training, images taken of chest radiographs can be used to confirm the presence or absence of diaphragmatic leveling, the possibility of inadequate expiratory breathing and hyperinflation in the lungs resulting in ineffective ventilation, and to adjust the frequency of training and set pressure. In addition, food oils and fats can be administered to a user as nutritional therapy in conjunction with training.

Next, an expiratory pressure load training management system of the present invention will be described.

The expiratory pressure load training management system of the present invention comprises a user terminal and a management server to which the expiratory pressure load training management device of the present invention described above is connected via a network. The user terminal and the management server share the functions of the expiratory pressure load training management device and are connected to each other via a network. A number of users enter their own exhalation information and information related to exercise tolerance via their own user terminals, are presented with a tailored expiratory pressure load training menu, and are presented with training results by entering trained exhalation information.

The functions of the user terminal and the management server will be described below.

The user terminal comprises the following 1a) to 1e) .
1a) An exhalation information input means including maximum expiratory muscle strength.
1b) A means for transmitting the input exhalation information to the management server.
1c) A menu presenting means for presenting a menu of expiratory pressure load training received from the management server.
1d) A means for inputting exhalation information after a predetermined number of expiratory pressure load training and information related to exercise tolerance and transmitting the same to the management server.
1e) A training result presenting means for presenting the training result received from the management server.

The management server comprises the following 2a) to 2d) .
2a) A means for receiving the above exhalation information from the user terminal.
2b) A user determination means for determining whether or not there is an exhalation prolongation during exercise as a result of calculating the difference on the basis of the received exhalation information.
2c) A menu creating means for creating and transmitting a menu of the expiratory pressure load training to the user terminal.
2d) A training result creating means for receiving the exhalation information after a predetermined number of expiratory pressure load training and information related to exercise tolerance from the user terminal, determining whether or not there is an effect of improving exercise tolerance of the user, creating a training result, and transmitting the training result to the user terminal.

Preferably, the user terminal is located either in a patient user's home, in a training facility, or in a medical clinic facility. Expiratory pressure load training can be easily performed at home, even in the elderly, and can also be performed in a training gym. Therefore, many user terminals are installed in the user's home, the training facility, and the medical clinic facility, and the management server for actually creating the training menu is centrally managed.

Next, an expiratory pressure load training management method of the present invention will be described.

The expiratory pressure load training management method is a training management method for a user having a COPD, and comprises the following steps a) to d).
a) An exhalation information input step for inputting exhalation information on the time ratio T_{EX} of inhalation or of exhalation to one breath time at peak exercise during CPET for a user, and the time ratio T_{RE} of inhalation or of exhalation to one breath time at rest during CPET.
b) A user determination step for calculating a difference between T_{EX} and T_{RE}, and determining whether or not the user has an exhalation prolongation during exercise.
c) A menu presenting step for presenting a menu of expiratory pressure load training based on a maximum expiratory muscle strength value to a user determined to have exhalation prolongation during exercise.
d) A training result presenting step for inputting the exhalation information and information related to exercise tolerance in a) above after a predetermined number of training sessions, and presenting a training result to determine whether or not there is an improvement effect of exercise tolerance of the user.

In the expiratory pressure load training management method of the present invention, expiratory pressure load training uses an expiratory pressure load device with a one-way valve, and the expiratory pressure load device opens when a set pressure is reached, and exhaled air flows in. Here, the set pressure can be set at a set range of low pressure less than or equal to 30cmH₂O, and can be trained and increased stepwise from a relatively low expiratory pressure load of 20% to 30% of based on the lower maximum expiratory muscle strength value of the users. In the user determination step, as a result of calculating the above-mentioned difference, if there is no exhalation prolongation during exercise, a menu of the inspiratory pressure load training is presented in the menu presenting step. The training menu in the menu presenting step is preferably confirmed and approved by the respiratory specialist.

Next, an expiratory pressure load training management program of the present invention will be described.

The expiratory pressure load training management program of the present invention is a computer program for causing a computer to function as all means provided in the user terminal in the expiratory pressure load training management system described above.

According to another aspect, the expiratory pressure load training management program of the present invention is a computer program for causing a computer to function as all means provided by the management server in the expiratory pressure load training management system described above.

According to another aspect, the expiratory pressure load training management program of the present invention is a computer program for causing a computer to execute an exhalation information input step, a user determination step, a menu presenting step, and a training result presenting step in the expiratory pressure load training management method described above.

### [Effects of the Invention]

According to the present invention, it is possible to reduce dyspnoea and improve exercise tolerance and physical activity in actual COPD patients.

### [Brief Description of the Drawings]

[FIG. 1] Figure. 1 shows a function block diagram of the expiratory pressure load training management device.
[FIG. 2] Figure. 2 shows a flow diagram of the expiratory pressure load training management method.
[FIG. 3] Figure. 3 shows a functional block diagram of the expiratory pressure load training management system.
[FIG. 4] Figure. 4 shows a configuration diagram of the expiratory pressure load training management system.
[FIG. 5] Figure. 5 shows graphs showing respiration timings and the volume of air remaining in the lung after expiration in one breathing, both of which are obtained from pre-training CPET for users with COPD.
[FIG. 6] Figure. 6 shows an improvement result of sustained exercise after expiratory pressure load training evaluated at the constant work rate exercise test.
[FIG. 7] Figure. 7 shows an improvement result of sustained exercise after inspiratory pressure load training evaluated at the constant work rate exercise test.
[FIG. 8] Figure. 8 shows a graph showing the effect of 3-month expiratory pressure load training (case presentation).
[FIG. 9] Figure. 9 shows an explanatory diagram of the ratio T of inspiratory time to one breathing time.

### [Best Mode for Carrying Out the Invention]

Embodiments of the present invention will be described in detail below with reference to the drawings. The present invention is not limited to the following embodiment and examples of shown in the figure, and the present invention can be variously changed in design.

### [Embodiment 1]

### (Expiratory pressure load training management device)

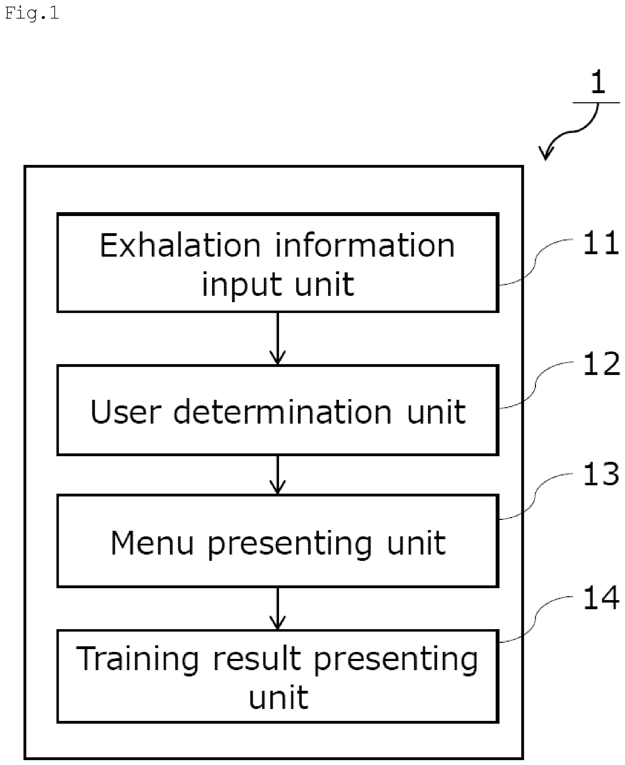

FIG. 1 shows a functional block diagram of the expiratory pressure load training management device of the embodiment 1. As shown in FIG. 1, the expiratory pressure load training management device 1 includes an exhalation information input unit 11, a user determination unit 12, a menu presenting unit 13, and a training result presenting unit 14. The exhalation information input unit 11 inputs the exhalation information about the ratio T_{EX} of the inhalation or of exhalation time to the one breath time at peak exercise during cardiopulmonary function test (CPET) to the user and the ratio T_{RE} of the inhalation or of exhalation time to the one breath time at the time of rest during CPET. The user determination unit 12 calculates a difference between T_{EX} and T_{RE}, and determines whether or not there is an exhalation prolongation during CPET. The menu presenting unit 13 presents a menu of expiratory pressure load training based on the maximum expiratory muscle strength value to the user who is determined to have exhalation prolongation during CPET as a result of calculating the difference. Further, the training result presenting unit 14 inputs exhalation information and information related to exercise tolerance after a predetermined number of times of training, determines whether or not there is an effect of improving exercise tolerance of the user, and presents the training result.

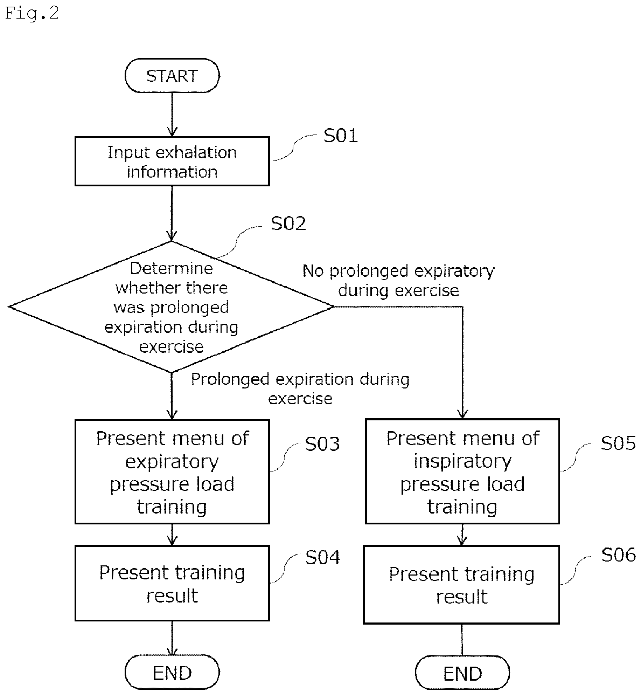

FIG. 2 shows a flow diagram of an expiratory pressure load training management method. As shown in FIG. 2, first, T_{EX} of the fraction of inspiratory or expiratory time to one breath time during cardiopulmonary exercise testing (CPET) for the user and T_{RE} of the fraction of inspiratory or of expiratory time to one breath time at rest during CPET and expiratory information are entered (Step S01: breath information entry step). Next, the difference between T_{EX} and T_{RE} is calculated, and it is determined whether or not the user has an exhalation prolongation during exercise (Step S02: User determination step).

In step S03, a menu of expiratory pressure load training is presented to a user who is determined to have an exhalation prolongation during exercise as a result of calculating the difference, based on the maximum expiratory muscle strength value. After the training is performed a predetermined number of times, exhalation information and information related to the exercise tolerance are input to determine whether or not there is an improvement effect of the exercise tolerance of the user, and the training result is presented in step S04.

In step S05, a menu of inspiratory pressure load training is presented based on the maximum inspiratory muscle strength value to the user who is determined as having no expiratory prolongation during exercise as a result of calculating the difference. After the training is performed a predetermined number of times, exhalation information and information related to the exercise tolerance are input to determine whether or not there is an improvement effect of the exercise tolerance of the user, and the training result is presented in step S06.

### [Embodiment 2]

### (Expiratory pressure load training management system)

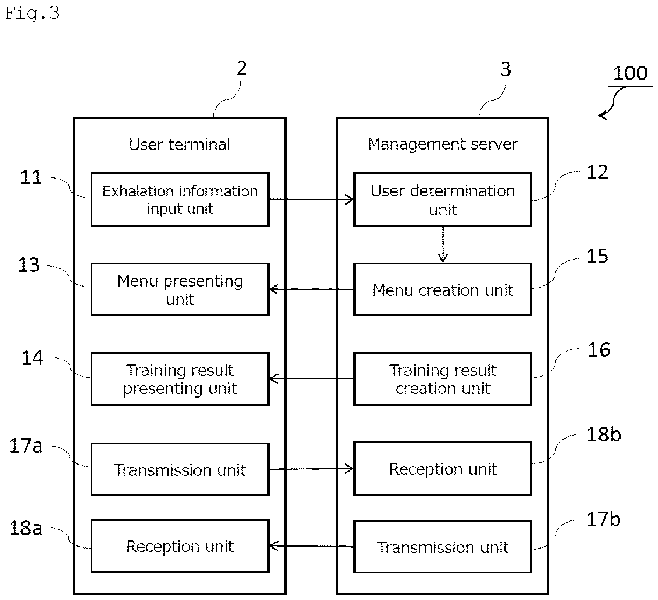

FIG. 3 shows a functional block diagram of an expiratory pressure load training management system. As shown in FIG. 3, the expiratory pressure load training management system 100 comprises a user terminal 2 and a management server 3, the user terminal 2 is provided with an exhalation information input unit 11, a menu presenting unit 13, a training result presenting unit 14, a transmission unit 17a, and a reception unit 18a, and the management server 3 is provided with a user determination unit 12, a menu creation unit 15, a training result creation unit 16, a transmission unit 17b, and a reception unit 18b.

The exhalation information input to the exhalation information input means of the user terminal 2 is sent to the management server 3 by the transmission unit 17a, and is received by the reception unit 18b provided in the management server 3. The user determination unit 12 determines whether or not the user has an exhalation prolongation as a result of calculating the difference based on the received exhalation information. The menu creation unit 15 creates a menu of the expiratory pressure load training or the inspiratory pressure load training based on the maximum expiratory muscle strength value or maximum inspiratory muscle strength value, respectively and transmits it by the transmission unit 17b. Information about the transmitted menu is received by the reception unit 18a provided in the user terminal 2. The menu presenting unit 13 provided in the user terminal 2 presents a menu of the expiratory pressure load training received from the management server 3.

The user terminal 2 inputs the exhalation information and the information related to the exercise tolerance after a predetermined number of training times by the transmission unit 17a, and transmits them to the management server 3. The management server 3 receives the exhalation information and the information related to the exercise tolerance from the user terminal 2 by the reception unit 18b. The management server 3 generates a training result by determining whether or not there is an improvement effect of the exercise tolerance of the user on the basis of exhalation information and information related to the exercise tolerance after a predetermined number of training sessions by the training result creation unit 16, transmits the training result to the user terminal 2 by the transmission unit 17b, and receives the training result in the reception unit 18a provided in the user terminal 2. The user terminal 2 presents the training result received from the management server 3 by the training result presenting unit 14.

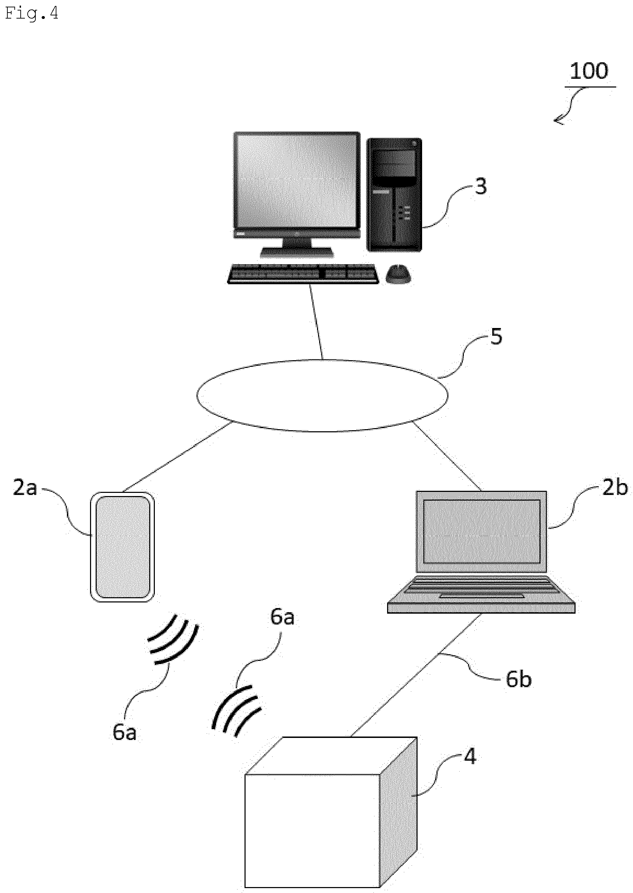

FIG. 4 is a block diagram of an expiratory pressure load training management system. As shown in FIG. 4, the expiratory pressure training management system 100 includes a smartphone 2a, a PC 2b, and a management server 3, and the smartphone 2a, a PC 2b, and the management server 3 are connected to each other via the Internet 5. Here, only the smartphone 2a and PC 2b are displayed as the user terminal 2, but in practice, a larger number of the user terminal 2 can be connected. The type of the user terminal 2 is not limited to a smartphone or a PC, and may be, for example, a tablet terminal or a wearable terminal.

An exercise load cardiopulmonary function test device 4 is provided with an exercise load device, an expiratory gas analyzer, and the like (not shown), and can perform tests for measuring O₂ concentration, CO₂ concentration, and the ventilatory flow during exercise in real time, and for measuring an index such as the peak oxygen uptake, which is an index of exercise tolerance.

In the present embodiment, the exercise load cardiopulmonary function test device 4 and the smartphone 2a communicate with each other by the wireless communication unit 6a, and the exercise load cardiopulmonary function test device 4 and PC 2b communicate with each other by the wired communication unit 6b, but the communication method is not limited to this, and for example, the exercise load cardiopulmonary function test device 4 and the smartphone 2a may communicate with each other by the wired communication unit 6b, and the exercise load cardiopulmonary function test device 4 and PC 2b may communicate with each other by the wireless communication unit 6a.

Alternatively, without using the wireless communication unit 6a or the wired communication unit 6b, for example, numerical values or the like displayed on a display (not shown) provided in the exercise load cardiopulmonary function test device 4 may be checked and inputted to the smartphone 2a or PC 2b.

### [Embodiment 3]

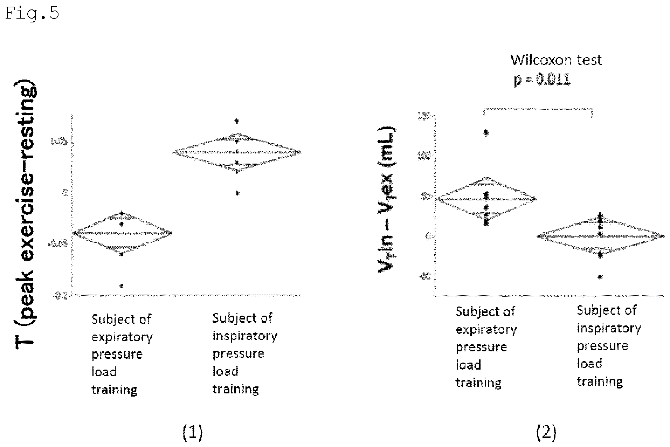

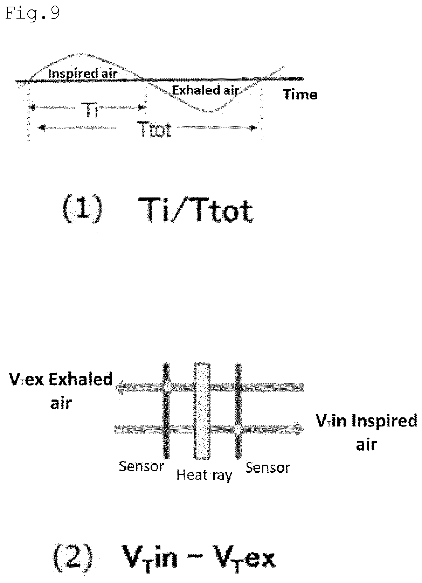

The results of confirming the usefulness of the expiratory pressure load training management device of the present invention will be described with respect to seven target users of the expiratory pressure load training and nine target users of the inspiratory pressure load training. FIG. 5(1) shows the difference T between the ratio T_{EX} at peak exercise and the ratio T_{RE} at rest in the ratio of the inspiratory time to one breath obtained from CPET prior to the intervention of the expiratory/inspiratory pressure load training. Moreover, FIG. 5(2) shows the volume of air remaining in the lung after expiration in one breathing (inspiratory V_{T}in - expiratory V_{T}ex) obtained from CPET prior to the intervention of the expiratory/inspiratory pressure load training. Here, the "Ti/Ttot" shown in FIG. 9(1) is the ratio of the inhalation time to one breath time, and the "V_{T}in-V_{T}ex" shown in FIG. 9(2) shows the remaining volume in the lung after the exhalation.

As shown in FIG. 5(1), T means the ration inspiratory time to total one breathing time. A user who experienced prolonged exhalation during exercise, i.e., a user who experienced a negative difference T (maximum exercise-resting) in the T-Low group, was the subject of expiratory pressure load training, while a patient who did not experience prolonged exhalation, i.e., a patient who experienced a positive difference T (maximum exercise-resting) in the T-Increase group, was the subject of inspiratory pressure load training.

As shown in FIG. 5(2), in CPET conducted prior to the training interventions, the volume of air remaining in the lung after expiration in one breathing at peak exercise, that is, the difference between the inspiratory tidal volume (V_{T}in) and the expiratory tidal volume (V_{T}ex) was significantly larger in the expiratory pressure load training subject than in the inspiratory pressure load training subject. In other words, it is suggested that the expiratory pressure load training subjects (T-lowering group) cannot completely exhale air by breathing once, and air accumulates in the lungs, which causes exertional dyspnoea, and how much air can be expelled is important in this group. Therefore, it can be said that if the expiratory pressure load training subject (T-lowering group) is subjected to expiratory pressure load training and sufficient exhalation and associated improvement in exhalation prolongation are obtained, it may lead to improvement in exercise tolerance. On the other hand, the person to be trained for inspiratory pressure load (T-increased group) has the ability to exhale air accumulated in the lungs during exercise. Therefore, even if the inspiratory ability is maintained and the inspiratory volume is increased, the load on exhaled air is considered to be small, and if sufficient ventilation is obtained by the inspiratory pressure load training, the exercise tolerance may improve.

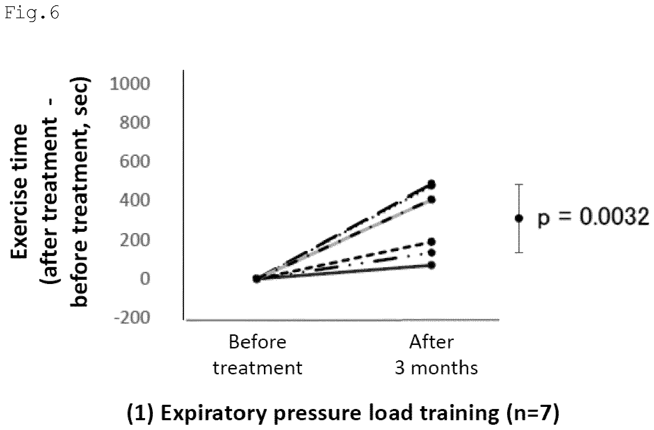

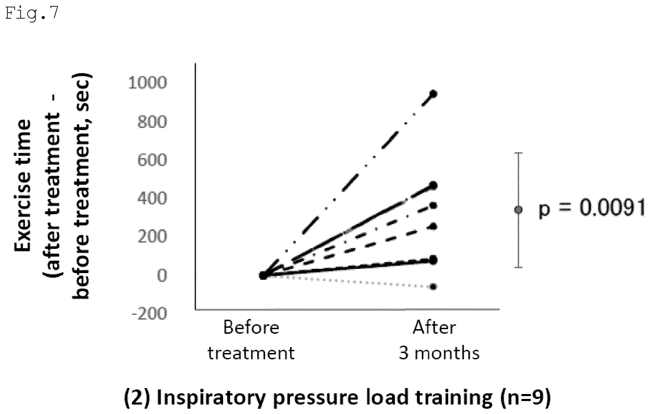

In fact, the results of evaluating the endurance time during the constant work rate exercise test after 3 months of training are shown in FIG. 6 and FIG. 7. FIG. 6 is an improvement result (n=7) after the expiratory pressure load training, and FIG. 7 is an improvement result (n=9) after the inspiratory pressure load training evaluated.

The improvement results of the constant work rate exercise test after the expiratory pressure load training shown in FIG. 6 was that the average exercise time before the training was 454 seconds (standard deviation 211 seconds) and the average exercise time after the training was 764 seconds (standard deviation 313 seconds), and it was confirmed that the change in exercise time from the pre-training was remarkably improved. Also, in the improvement result of the constant work rate exercise test after the inspiratory pressure load training shown in FIG. 7, the average exercise time before the training was 458 seconds (standard deviation 203 seconds), but the average exercise time after the training was 799 seconds (standard deviation 495 seconds), which also confirmed the remarkable improvement of the exercise time. In particular, it has been an interesting result that users who have undergone expiratory pressure load training have achieved significant improvement despite the fact that adequate inhalation therapy has already been introduced and that many users have undergone pulmonary rehabilitation to date.

### [Embodiment 4]

### (Actual case of COPD: Exercise times and exertional dyspnoea improved by expiratory pressure load training)

The case of a person with COPD is illustrated. The patient was a 64-year-old man with a height of 174cm, weight of 69kg, and no past other medical history (e.g., asthma).

Historical treatment with three COPD long-acting anticholinergics, long-acting β-agonists, and inhaled corticosteroids resulted in a plateau gait of at most 200m because of exertional dyspnoea. Non-pharmacological therapies such as pulmonary rehabilitation had been added, but his condition was insufficient. Therefore, we participated in a clinical study including expiratory pressure loading training. The subject was diagnosed with a markedly decreased pulmonary function in spite of inhalation of the three inhaled drugs described above, with a 1-second dose (FEV₁), 0.89L, %FEV₁, 26.9%, and COPD stage IV: most severe (staging divided into four stages I-IV) in the preliminary assessment of respiratory pressure load training. He also participated in a clinical study to determine whether expiratory and inspiratory pressure load training should be performed according to the degree of expiratory prolongation during exercise, and when the ratio of inspiratory time to one respiratory time during exercise was confirmed by CPET using an ergometer, the proportion of inspiratory time decreased (the expiratory time increased) as he exercised, so that insufficient exhalation was considered to be related to exertional dyspnoea, and an expiratory pressure load training menu was presented.

In the examination opinion using the thorax roentgen, the diaphragm was flattened, and the possibility in which the exhalation was insufficient and hyperinflation in the lung and it fell into the wasted ventilation was indicated.

The expiratory pressure load training presented is as follows.

The training (30 times a set, two sets, e.g. morning, evening, a day) was performed for 12 weeks at home. The training load was started at 20% of the maximum expiratory pressure at baseline. Thereafter, the level was increased once every 2 weeks up to 50% of the maximum pressure. This case reached the target of 50%, and the training was finished.

The overall outcome was as follows. In pulmonary function tests, after 3 months of expiratory pressure loading training, some improvement was obtained as shown in Table 1 below. In Table 1, FEV₁ indicates the amount of 1 second, and the VC indicates the vital capacity.

**[Table 1]**

| Pulmonary function | Before training | After 3 months of training |
|---|---|---|
| FEV₁, (L) | 0.89 | 0.93 |
| %FEV₁,(%) | 26.9 | 28.1 |
| VC,(L) | 3.65 | 3.81 |

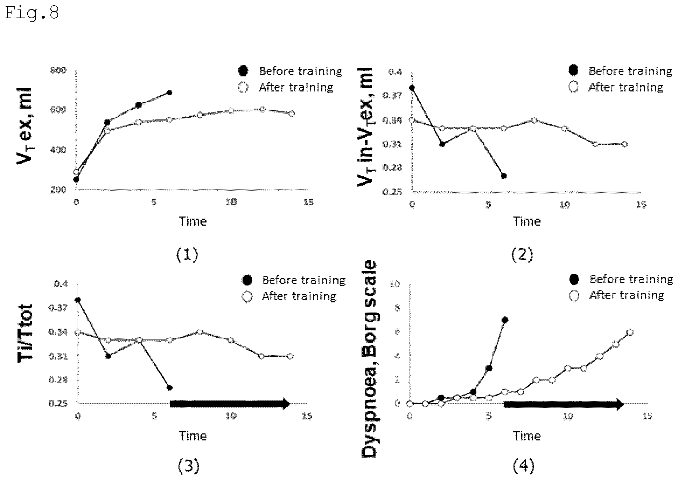

In incremental CPET, which comprised 2-minute increments to 10W, the minute ventilation increased by increasing the tidal volume after expiratory pressure load training, resulting in increased O₂ uptake and improved exercise tolerance. Interestingly, the constant work rate exercise test, which was performed at 70% of the peak work load in the incremental exercise test before training, resulted in significant prolongation of exercise time (see Table 2 below and FIG. 8).

As shown in FIG. 8, even though the expiratory tidal volume decreased on the constant work rate exercise test after the expiratory pressure load training, the volume of air remaining in the lung after expiration in one breathing decreased, that is, sufficient exhaled air was possible, and the exercise was terminated without decreasing the ratio T of the inspiratory time to the one breath during exercise, that is, without prolonging the expiratory time. As a result, the inclination of the Borg scale, which represents the slope of dyspnoea during exercise, is gradual, and the prolongation of exercise time is about 9 minutes, which is a surprising effect.

The Borg scale represents the degree of exertional dyspnoea in steps 0 to 10, and the peak oxygen uptake for exercise tolerance positively correlates with the minute ventilation. Here, the minute ventilation is calculated as the product of the tidal volume and the respiration rate.

**[Table 2]**

| During maximum exertion | Before training | After training |
|---|---|---|
| Dyspnoea, Borg scale | 8 | 7 |
| Peak oxygen uptake, ml/min | 632 | 725 |
| Minute ventilation, L/min | 28.5 | 31.1 |
| Expiratory tidal volume, ml | 1132 | 1193 |
| Respiratory frequency, /min | 25 | 26 |

### [Industrial Applicability]

The present invention is useful in the expiratory pressure load training management device.

### [Description of Symbols]

- 1: Expiratory pressure load training management system
- 2: User terminal
- 2a: Smartphone
- 2b: PC
- 3: Management server
- 4: Exercise stress cardiopulmonary function testing device
- 5: Internet
- 6a: Wireless Communication Means
- 6b: Wired Communication Means
- 11: Exhalation information input unit
- 12: User determination unit
- 13: Menu presenting unit
- 14: Training result presenting unit
- 15: Menu creation unit
- 16: Training result creation unit
- 17a, 17b: Transmission unit
- 18a, 18b: Reception unit

## Claims

1. An expiratory pressure load training management device, which is a training management device for a user with chronic obstructive pulmonary disease (COPD), comprising:
an exhalation information input means for inputting exhalation information on the time ratio T_{EX} of inhalation or of exhalation to one breath time at peak exercise during cardiopulmonary exercise test (CPET) for a user, and the time ratio T_{RE} of inhalation or of exhalation to one breath time at rest during CPET;
a user determination means for calculating a difference between T_{EX} and T_{RE}, and determining whether or not the user has exhalation prolongation during exercise;
a menu presenting means for presenting a menu of expiratory pressure load training based on a maximum expiratory muscle strength value to a user determined to have exhalation prolongation during exercise; and
a training result presenting means for inputting the exhalation information and information related to exercise tolerance after a predetermined number of training sessions, and presenting a training result to determine whether or not there is an improvement effect of exercise tolerance of the user.

2. The expiratory pressure load training management device according to claim 1, wherein the expiratory pressure load training uses an expiratory pressure load device with a one-way valve, and the device opens the valve when a set pressure is reached, and exhaled air flows in.

3. The expiratory pressure load training management device according to claim 2, wherein the set pressure is settable at a set range of less than or equal to 30cmH₂O, and can be trained and increased in a stepwise manner from an expiratory pressure load of 20-30% of the low maximum expiratory muscle strength value of the users.

4. The expiratory pressure load training management device according to any one of claims 1 to 3, wherein the menu presenting means presents a menu of inspiratory pressure load training, when the user determination means is no exhalation prolongation during exercise as a result of the exertional change in the time ratio of inhalation or exhalation to one breath time between during exercise and at rest.

5. The expiratory pressure load training management device according to any one of claims 1 to 4, wherein the menu in the menu presenting means is confirmed and approved by a respiratory specialist.

6. An expiratory pressure load training management system comprising:
a user terminal and a management server connected via a network that function as the expiratory pressure load training management device according to any one of claims 1 to 5,
wherein the user terminal is the exhalation information input means, means for transmitting the input exhalation information to the management server, menu presenting means for presenting a menu of the expiratory pressure load training received from the management server, means for inputting and transmitting to the management server information relating to the exhalation information and exercise tolerance after a predetermined number of training sessions, and training result presenting means for presenting the training result received from the management server, and
wherein the management server is a means for receiving the exhalation information and information relating to exercise tolerance from a user terminal, the user determination means for determining whether or not there is an exhalation prolongation during exercise as a result of calculating the difference on the basis of the received exhalation information, a menu creating means for creating a menu of the expiratory pressure load training and transmitting it to a user terminal, and a training result creating means for receiving the exhalation information and information related to the exercise tolerance after a predetermined number of training sessions from a user terminal, determining whether or not there is an improvement effect of the exercise tolerance of the user, creating a training result, and transmitting the training result to the user terminal.

7. The expiratory pressure load training management system according to claim 6, wherein the user terminal is located either in a patient user's home, in a training facility, or in a medical clinic facility.

8. An expiratory pressure load training management method, which is a training management method for a user with chronic obstructive pulmonary disease (COPD), comprising:
an exhalation information input step for inputting exhalation information on the time ratio T_{EX} of inhalation or of exhalation to one breath time at peak exercise during cardiopulmonary exercise test (CPET) for a user, and the time ratio T_{RE} of inhalation or of exhalation to one breath time at rest during CPET;
a user determination step for calculating a difference between T_{EX} and T_{RE}, and determining whether or not the user has exhalation prolongation during exercise;
a menu presenting step for presenting a menu of expiratory pressure load training based on a maximum expiratory muscle strength value to a user determined to have exhalation prolongation during exercise; and
a training result presenting step for inputting the exhalation information and information related to exercise tolerance after a predetermined number of training sessions, and presenting a training result to determine whether or not there is an improvement effect of exercise tolerance of the user.

9. The expiratory pressure load training management method according to claim 8, wherein the expiratory pressure load training uses an expiratory pressure load device with a one-way valve, and the device opens the valve when a set pressure is reached, and exhaled air flows in.

10. The expiratory pressure load training management method according to claim 9, wherein the set pressure is settable at a set range of less than or equal to 30cmH₂O, and can be trained and increased in a stepwise manner from an expiratory pressure load of 20-30% of the maximum expiratory muscle strength value of the users.

11. The expiratory pressure load training management method according to any one of claims 8 to 10, wherein the menu presenting means presents a menu of inspiratory pressure load training, when the user determination means is no exhalation prolongation during exercise as a result of the exertional change in the time ratio of inhalation or of exhalation to one breath time between during exercise and at rest.

12. The expiratory pressure load training management method according to any one of claims 8 to 11, wherein the menu in the menu presenting means is confirmed and approved by a respiratory specialist.

13. A computer program for causing a computer to function as all means provided in the user terminal in the expiratory pressure load training management system according to claim 6.

14. A computer program for causing a computer to function as all means provided in the management server in the expiratory pressure load training management system according to claim 6.

15. A computer program for causing a computer to execute the exhalation information input step, the user determination step, the menu presenting step, and the training result presenting step in the expiratory pressure load training management method according to any one of claims 8 to 12.
